# EUROPEAN PATENT SPECIFICATION

(11) **EP 1 509 273 B1**
(45) Date of publication and mention of the grant of the patent: **07.01.2009**
(21) Application number: 03728627.5
(22) Date of filing: 30.04.2003
(51) Int. Cl.: A61M 25/01, A61M 25/00

(54) **RADIOPAQUE AND MRI COMPATIBLE CATHETER BRAID**
STRAHLUNGSUNDURCHLÄSSIGES UND MAGNETRESONANZKOMPATIBLES KATHETERGEFLECHT
TRESSE DE CATHETER RADIO-OPAQUE ET COMPATIBLE AVEC L'IRM

(30) Priority: 16.05.2002 US 146980
(43) Date of publication of application: 02.03.2005
(73) Proprietor: Boston Scientific Limited, St. Michael, Barbados, West Indies (BB)
(72) Inventor: PEPIN, Henry, J., Loretto, MN 55357 (US); WILLARD, Martin, R., Burnsville, Minnesota 55337 (US); PU, Zhou, Maple Grove, MN 55311 (US); KAMPA, Greg, J., Blaine, MN 55434 (US)
(74) Representative: Vossius & Partner
(86) International application number: PCT/US2003/013535
(87) International publication number: WO 2003/097148

(56) References cited:
- EP-A- 0 810 003
- US-A- 5 728 079
- US-A- 5 947 940

## Description

The present invention generally relates to catheter shafts. More specifically, the present invention relates to reinforced catheter shafts for intravascular devices such as guide catheters, diagnostic catheters, balloon catheters, and the like. See for example US-A-5 728 079.

Diagnostic catheters and guide catheters are commonly used to facilitate the diagnosis and treatment of vascular diseases such as coronary artery disease and peripheral vascular disease. Such catheters commonly include a braid reinforcement layer disposed between an inner layer and an outer layer. The braid reinforcement provides torsional rigidity, column strength, kink resistance, as well as radiopacity. However, conventional braid reinforcement materials such as stainless steel are not MRI (magnetic resonance imaging) compatible due to ferro-magnetic properties. Because different visualization techniques may be employed to facilitate intravascular navigation, it is desirable to have a catheter shaft that is both radiopaque for x-ray visualization and non-magnetically responsive for MRI compatibility.

To address these desirable features, the present invention provides, for example, an intravascular catheter comprising a reinforced shaft that is entirely non-magnetically responsive and at least partially radiopaque. In one specific example and without limitation, the present invention provides an elongate catheter shaft that is entirely non-magnetically responsive and at least partially radiopaque, wherein the shaft includes an inner layer, an outer layer, a reinforcement layer disposed between the inner and outer layers, and a soft distal tip. The reinforcement layer may comprise a braid of non-magnetically responsive radiopaque metal wires, the outer layer may comprise a non-radiopaque flexible polymer, the inner layer may comprise a non-radiopaque lubricious polymer, and the soft distal tip may comprise a polymer loaded with a radiopaque non-magnetically responsive filler. The inner layer, the outer layer and the reinforcement layer may extend from the proximal end of the shaft to the proximal end of the distal tip, leaving the tip flexible and atraumatic.
Figure 1 is a plan view of an intravascular catheter in accordance with an embodiment of the present invention, shown as a guide or diagnostic catheter,
Figure 2 is a cross-sectional view taken along line 2-2 in Figure 1;
Figure 3 is a longitudinal sectional view taken along line 3-3 in Figure 1;
Figures 4A - 4C are fragmentary views of various braid options; and
Figure 5 is a partially sectioned fragmentary view of the catheter shaft shown in Figure 1.

The following detailed description should be read with reference to the drawings in which similar elements in different drawings are numbered the same. The drawings, which are not necessarily to scale, depict illustrative embodiments and are not intended to limit the scope of the invention.

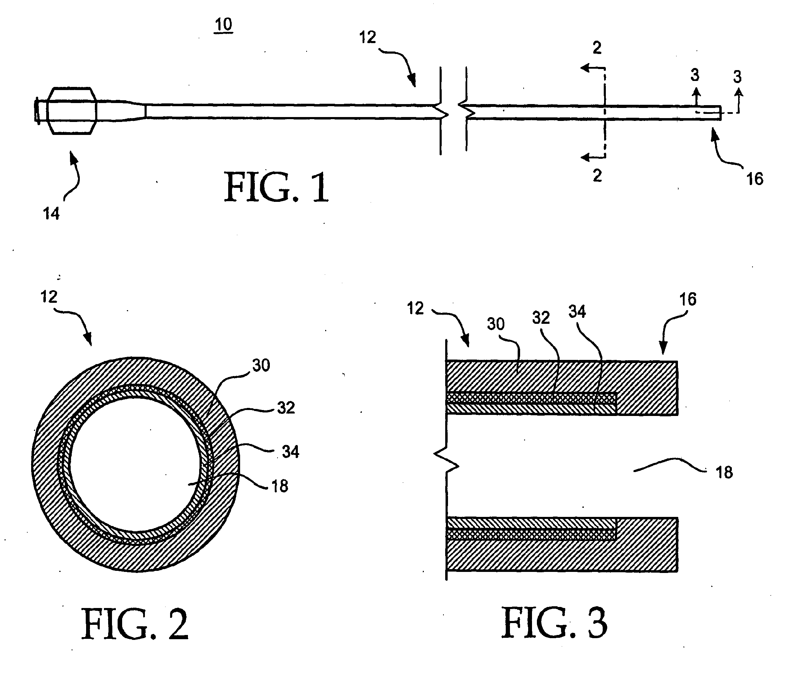

Refer now to Figure 1 which illustrates an intravascular catheter in accordance with an embodiment of the present invention. For purposes of illustration and discussion only, the intravascular catheter shown in Figure 1 is in the form of a guide or diagnostic catheter 10, but may comprise virtually any catheter used for intravascular applications. For example, the intravascular catheter may comprise a balloon catheter, an atherectomy catheter, a drug delivery catheter, a stent delivery catheter, etc.

As used herein, magnetically non-responsive materials refer to materials that are compatible with magnetic resonance imaging techniques. By way of example, not limitation, non-magnetically responsive materials include materials with a magnetic susceptibility (absolute value) less than 1×10⁻⁴, preferably less than 1×10⁻⁵, and ideally near zero (0). By contrast, magnetically responsive materials include materials with a magnetic susceptibility (absolute value) greater than or equal to 1×10⁻⁴.

Generally speaking, polymers and some metals such as Titanium are magnetically non-responsive, and metals such as stainless steel and other ferrous containing metals are magnetically responsive.

The guide or diagnostic catheter 10 may have a length and an outside diameter sufficient to enable intravascular insertion and navigation. For example, the catheter 10 may have a length of approximately 100cm-150cm and an outside diameter of approximately 4F-9F. The guide or diagnostic catheter 10 may be substantially conventional except as described herein and shown in the drawings.

The catheter 10 includes an elongate shaft 12 having a proximal end and distal end. A distal tip 16 is connected to the distal end of the elongate shaft 12. The distal tip 16 and a distal portion of the elongate shaft 12 may be curved depending on the particular clinical application. The elongate shaft 12 and the distal tip 16 include a lumen 18 extending therethrough to facilitate insertion of other medical devices (e.g., guide wires, balloon catheters, etc.) therethrough, and/or to facilitate injection of fluids (e.g., radiopaque dye, saline, drugs, etc.) therethrough. A conventional manifold 14 is connected to the proximal end of the elongate shaft 12 to facilitate connection to other medical devices (e.g., syringe, Y-adapter, etc.) and to provide access to the lumen 18.

As best seen in Figures 2 and 3, the elongate shaft 12 may be multi-layered. In this embodiment, the elongate shaft 12 may include an outer layer 30, a reinforcement layer 32, and an inner layer 34.. The distal tip 16 may comprise the outer layer 30 extending beyond the inner layer 34 and the reinforcement layer 32 to define a soft atraumatic tip.

The inner layer 34 may comprise a lubricious polymer such as HDPE or PTFE, for example. In one particular embodiment, the inner layer 34 may comprise PTFE having a wall thickness of 0,025 mm (0.001 in.), and an inside diameter of 1,473 mm (0.058 inches). In this example, the inner layer 34 is non-magnetically responsive and non-radiopaque, but may be made radiopaque by utilizing known filler materials such as bismuth subcarbonate.

The outer layer 30 may comprise, at least in part, a polyether-ester elastomer sold under the trade name ARNITEL. The outer layer 30 may be formed, for example, by extrusion, co-extrusion, interrupted layer co-extrusion (ILC), or fusing several segments end-to-end. The outer layer may have a uniform stiffness or a gradual reduction in stiffness from the proximal end to the distal end thereof. The gradual reduction in stiffness may be continuous as by ILC or may be stepped as by fusing together separate extruded tubular segments end-to-end. The outer layer may be impregnated with a reinforcing material such as liquid crystal polymer (LCP) fibrils. For example, a proximal portion of the outer layer 30 may comprise 74D ARNITEL with 6% LCP, a mid portion may comprise 63D ARNITEL, and a distal portion may comprise 55D ARNITEL. The distal tip 16 may comprise 40D ARNITEL loaded with 46% bismuth subcarbonate to render it radiopaque. The proximal portion, mid portion, distal portion and distal tip may have lengths of 863 mm (34 in), 76 mm (3 in.), 38 mm (1.5 in), and 3,8 mm (0.15 in.), respectively. The proximal, mid and distal portions may have a wall thickness of 0.127 mm (0.005 in.), and the distal tip may have a wall thickness of 0,127 mm (0.005 in). In this example, the outer layer 30 is non-radiopaque and non-magnetically responsive, while the distal tip 16 is radiopaque and non-magnetically responsive.

The reinforcement layer 32 may comprise a metal wire braid, for example. The metal wire braid may comprise a non-magnetically responsive (i.e., non-ferrous) radiopaque metal such as Tungsten, Gold, Titanium, Silver, Copper, Platinum, Iridium, other non-ferrous dense metals, or alloys thereof. Tungsten exhibits tensile properties (strength and rigidity) similar to or higher than those of stainless steel, which is a conventional reinforcement material that exhibits magnetic responsiveness due to some ferrous content and is therefore not MRI compatible. Tungsten is also relatively dense and therefore relatively radiopaque. Tungsten is also relatively inexpensive compared to other more precious metals and alloys.

Alternatively, the reinforcement layer 32 may be formed of a non-metal material such as poly-para-phenylene terephthalamide (KEVLAR) fibers, LCP fibers, other polymeric filaments, or glass fibers, including monofilament and multi-filament structures of each.

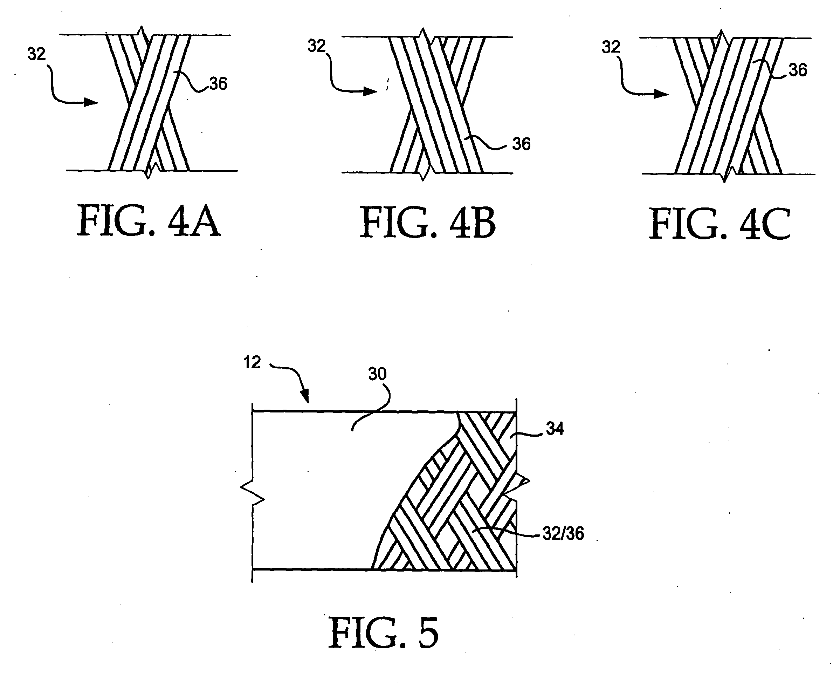

As seen in Figures 4A - 4C, the braid reinforcement layer 32 may comprise one or more strands 36 of non-magnetically responsive (i.e., non-ferrous) radiopaque material. Each strand 36 may be flat (ribbon), round, and/or hollow. By way of example, not limitation, the braid 32 may include triple strands 36 braided in a three-over-three pattern as seen in Figure 4A, quadruple strands 36 braided in a four-over-four pattern as seen in Figure 4B, or quintuple strands 36 braided in a five-over-five pattern as shown in Figure 4C. As seen in Figure 5, a triple strand (three-over-three) reinforcement braid 32 utilizing 0,025 mm (0.001 inch) diameter Tungsten wire strands 36 with a pic count (pic count refers to the number intersections between strand sets per lineal unit) of 1650 +/- 127 pics/mm (66 +/- 5 pics/inch) has been found to provide good radiopacity without requiring loading of the outer layer 30, and good shaft 12 performance in terms of kink resistance, torque transmission, pushability, and shape retention.

## Claims

1. An intravascular catheter comprising an elongate shaft that is entirely non-magnetically responsive and at least partially radiopaque, **characterized in that** the elongate shaft including a reinforcement layer comprising non-magnetically responsive radiopaque metal wires, wherein the elongate shaft has a magnetic susceptibility in absolute value less than 1×10⁻⁴ and wherein the elongate shaft includes an inner layer and an outer layer with the reinforcement layer disposed therebetween, the outer layer comprising a non-radiopaque polymer and the inner layer comprising a non-radiopaque lubricious polymer.

2. An intravascular catheter as in claim 1, wherein the elongate shaft has a magnetic susceptibility in absolute value less than 1×10⁻⁵.

3. An intravascular catheter as in claim 1, wherein the elongate shaft has a magnetic susceptibility in absolute value near zero (0).

4. An intravascular catheter as in claim 1, wherein the elongate shaft includes a soft distal tip comprising a polymer loaded with a radiopaque non-magnetically responsive filler.

5. An intravascular catheter as in claim 4, wherein the inner layer, the outer layer and the reinforcement layer extend from a proximal end of the shaft to the distal tip.

6. An intravascular catheter as in claim 1, wherein the non-magnetically responsive radiopaque metal comprises Tungsten.

7. An intravascular catheter as in claim 4, wherein the reinforcement layer comprises a braid.

8. An intravascular catheter as in claim 7, wherein the braid comprises multiple strands.

9. An intravascular catheter as in claim 8, wherein the multiple strands are braided in a three-over-three pattern.

10. An intravascular catheter as in claim 8, wherein the multiple strands are braided in a four-over-four pattern.

11. An intravascular catheter as in claim 8, wherein the multiple strands are braided in a five-over-five pattern.

12. An intravascular catheter as in claim 7, wherein the elongate shaft further comprises an inner layer and an outer layer with the reinforcement layer disposed therebetween.

13. An intravascular catheter as in claim 12, wherein the elongate shaft further comprises a soft distal tip.

14. An intravascular catheter as in claim 13, wherein the inner layer, the outer layer and the reinforcement layer extend from a proximal end of the shaft to a proximal end of the distal tip.

15. An intravascular catheter as in claim 14 wherein the reinforcement layer comprises a plurality of non-magnetically responsive radiopaque metal wires.

16. An intravascular catheter as in claim 15, wherein the outer layer comprises a non-radiopaque polymer.

17. An intravascular catheter as in claim 16, wherein the outer layer is non-radiopaque.

18. An intravascular catheter as in claim 17, wherein the inner layer comprises a non-radiopaque lubricious polymer.

19. An intravascular catheter as in claim 18, wherein the distal tip comprises a polymer loaded with a radiopaque non-magnetically responsive filler.

## Patentansprüche

1. Intravaskulärer Katheter mit einem langgestreckten Schaft, der völlig nichtmagnetisch reagiert und zumindest partiell strahlenundurchlässig ist, **dadurch gekennzeichnet, daß** der langgestreckte Schaft eine Verstärkungsschicht mit nichtmagnetisch reagierenden, strahlenundurchlässigen Metalldrähten aufweist, wobei der langgestreckte Schaft eine magnetische Suszeptibilität im Absolutwert kleiner als 1 x 10⁻⁴ hat und wobei der langgestreckte Schaft eine Innenschicht und eine Außenschicht mit der dazwischen befindlichen Verstärkungsschicht aufweist, wobei die Außenschicht ein strahlendurchlässiges Polymer aufweist und die Innenschicht ein strahlendurchlässiges Gleitpolymer aufweist.

2. Intravaskulärer Katheter nach Anspruch 1, wobei der langgestreckte Schaft eine magnetische Suszeptibilität im Absolutwert kleiner als 1 x 10⁻⁵ hat.

3. Intravaskulärer Katheter nach Anspruch 1, wobei der langgestreckte Schaft eine magnetische Suszeptibilität im Absolutwert nahe Null (0) hat.

4. Intravaskulärer Katheter nach Anspruch 1, wobei der langgestreckte Schaft eine weiche distale Spitze mit einem Polymer hat, das mit einem strahlenundurchlässigen, nichtmagnetisch reagierenden Füllstoff versehen ist.

5. Intravaskulärer Katheter nach Anspruch 4, wobei sich die Innenschicht, die Außenschicht und die Verstärkungsschicht von einem proximalen Ende des Schafts zur distalen Spitze erstrecken.

6. Intravaskulärer Katheter nach Anspruch 1, wobei das nichtmagnetisch reagierende, strahlenundurchlässige Metall Wolfram aufweist.

7. Intravaskulärer Katheter nach Anspruch 4, wobei die Verstärkungsschicht ein Geflecht aufweist.

8. Intravaskulärer Katheter nach Anspruch 7, wobei das Geflecht mehrere Stränge aufweist.

9. Intravaskulärer Katheter nach Anspruch 8, wobei die mehreren Stränge in einem Drei-auf-drei-Muster geflochten sind.

10. Intravaskulärer Katheter nach Anspruch 8, wobei die mehreren Stränge in einem Vier-auf-vier-Muster geflochten sind.

11. Intravaskulärer Katheter nach Anspruch 8, wobei die mehreren Stränge in einem Fünf-auf-fünf-Muster geflochten sind.

12. Intravaskulärer Katheter nach Anspruch 7, wobei der langgestreckte Schaft ferner eine Innenschicht und eine Außenschicht mit der dazwischen befindlichen Verstärkungsschicht aufweist.

13. Intravaskulärer Katheter nach Anspruch 12, wobei der langgestreckte Schaft ferner eine weiche distale Spitze aufweist.

14. Intravaskulärer Katheter nach Anspruch 13, wobei sich die Innenschicht, die Außenschicht und die Verstärkungsschicht von einem proximalen Ende des Schafts zu einem proximalen Ende der distalen Spitze erstrecken.

15. Intravaskulärer Katheter nach Anspruch 14, wobei die Verstärkungsschicht mehrere nichtmagnetisch reagierende, strahlenundurchlässige Metalldrähte aufweist.

16. Intravaskulärer Katheter nach Anspruch 15, wobei die Außenschicht ein strahlendurchlässiges Polymer aufweist.

17. Intravaskulärer Katheter nach Anspruch 16, wobei die Außenschicht strahlendurchlässig ist.

18. Intravaskulärer Katheter nach Anspruch 17, wobei die Innenschicht ein strahlendurchlässiges Gleitpolymer aufweist.

19. Intravaskulärer Katheter nach Anspruch 18, wobei die distale Spitze ein Polymer aufweist, das mit einem strahlenundurchlässigen, nichtmagnetisch reagierenden Füllstoff versehen ist.

## Revendications

1. Cathéter intravasculaire comprenant un arbre allongé sans aucune susceptibilité magnétique et au moins partiellement radio-opaque, **caractérisé en ce que** cet arbre allongé intègre une couche de renforcement comprenant des fils métalliques radio-opaques sans susceptibilité magnétique,
dans lequel l'arbre allongé a une susceptibilité magnétique en valeur absolue inférieure à 1×10⁻⁴,
et dans lequel l'arbre allongé intègre une couche interne et une couche externe, la couche de renforcement étant disposée entre celles-ci, la couche externe comprenant un polymère non radio-opaque et la couche interne comprenant un polymère lubrifiant non radio-opaque.

2. Cathéter intravasculaire selon la revendication 1, dans lequel l'arbre allongé a une susceptibilité magnétique en valeur absolue inférieure à 1×10⁻⁵.

3. Cathéter intravasculaire selon la revendication 1, dans lequel l'arbre allongé a une susceptibilité magnétique en valeur absolue proche de zéro (0).

4. Cathéter intravasculaire selon la revendication 1, dans lequel l'arbre allongé intègre un embout distal souple comprenant un polymère chargé avec une charge radio-opaque sans susceptibilité magnétique.

5. Cathéter intravasculaire selon la revendication 4, dans lequel la couche interne, la couche externe et la couche de renforcement s'étendent depuis une extrémité proximale de l'arbre jusqu'à l'embout distal.

6. Cathéter intravasculaire selon la revendication 1, dans lequel le métal radio-opaque sans susceptibilité magnétique comprend du Tungstène.

7. Cathéter intravasculaire selon la revendication 4, dans lequel la couche de renforcement comprend une tresse.

8. Cathéter intravasculaire selon la revendication 7, dans lequel la tresse comprend des brins multiples.

9. Cathéter intravasculaire selon la revendication 8, dans lequel les brins multiples sont tressés selon un motif trois sur trois.

10. Cathéter intravasculaire selon la revendication 8, dans lequel les brins multiples sont tressés selon un motif quatre sur quatre.

11. Cathéter intravasculaire selon la revendication 8, dans lequel les brins multiples sont tressés selon un motif cinq sur cinq.

12. Cathéter intravasculaire selon la revendication 7, dans lequel l'arbre allongé comprend en outre une couche interne et une couche externe, la couche de renforcement étant disposée entre celles-ci.

13. Cathéter intravasculaire selon la revendication 12, dans lequel l'arbre allongé comprend en outre une extrémité distale souple.

14. Cathéter intravasculaire selon la revendication 13, dans lequel la couche interne, la couche externe et la couche de renforcement s'étendent depuis une extrémité proximale de l'arbre jusqu'à une extrémité proximale de l'embout distal.

15. Cathéter intravasculaire selon la revendication 14, dans lequel la couche de renforcement comprend une pluralité de fils métalliques radio-opaques sans susceptibilité magnétique.

16. Cathéter intravasculaire selon la revendication 15, dans lequel la couche externe comprend un polymère non radio-opaque.

17. Cathéter intravasculaire selon la revendication 16, dans lequel la couche externe est non radio-opaque.

18. Cathéter intravasculaire selon la revendication 17, dans lequel la couche interne comprend un polymère lubrifiant non radio-opaque.

19. Cathéter intravasculaire selon la revendication 18, dans lequel l'embout distal comprend un polymère chargé avec une charge radio-opaque sans susceptibilité magnétique.
